# EUROPEAN PATENT APPLICATION

(11) **EP 4 394 779 A1**
(43) Date of publication of application: **03.07.2024**
(21) Application number: 22873185.7
(22) Date of filing: 22.09.2022
(51) Int. Cl.: G16B 40/20, G16B 25/10, G16B 30/10, C12Q 1/6886, G01N 33/574, G16B 35/00, G16B 50/00

(54) **METHOD FOR DIAGNOSING CANCER OF UNKNOWN PRIMARY SITE BY USING ARTIFICIAL INTELLIGENCE**

(30) Priority: 24.09.2021 KR 20210126398; 16.05.2022 KR 20220059550
(71) Applicant: Oncocross Co., Ltd., Seoul 04168 (KR)
(72) Inventor: LEE, Young Heun, Seoul 07026 (KR); KIM, Yi Rang, Sejong 30064 (KR); KANG, Ji Hoon, Seoul 05532 (KR)
(74) Representative: BCKIP Part mbB
(86) International application number: PCT/KR2022/014191
(87) International publication number: WO 2023/048481

(57) **Abstract**

A method for diagnosing cancer of unknown primary site by using artificial intelligence is disclosed. A method for diagnosing cancer of unknown primary site by using artificial intelligence, according to one embodiment of the present invention, includes the steps of: generating gene expression pattern information of a sample collected from tissue in which metastatic cancer has occurred; removing gene expression pattern information derived from pre-learned tissue from the gene expression pattern information of the sample collected from the tissue in which the metastatic cancer has occurred; comparing the gene expression pattern information from which the gene expression pattern information derived from the tissue has been removed with pre-learned gene expression pattern information for each cancer type; and specifying a primary site of the sample collected from the tissue in which the metastatic cancer has occurred.

## Description

### BACKGROUND

### Field

The present invention relates to a method for diagnosing cancer of unknown primary site using artificial intelligence, and more particularly, to a method for diagnosing cancer of unknown primary site using artificial intelligence capable of further improving the accuracy of diagnosis by detecting the primary site of cancer using gene expression level information in tissue where cancer has metastasized, but excluding gene expression effects in metastatic tissue.

### Related Art

Cells, the smallest unit of the body, have their own order and self-regulating function to keep their number in balance. However, when the number of newly created cells exceeds that of dying cells with unknown cause, unnecessary extra cells do not perform their role properly and clump together in one place to settle down.

This form is called a tumor. The tumor in a state in which the tumor does not stop at a certain size and constantly proliferates and invades surrounding normal cells is defined as a malignant tumor, that is, cancer.

Cancer may be divided into primary cancer, in which cancer cell tissues first settle down and begin to be formed, and metastatic cancer, which is generated in other organs by moving cancer cells from the primary organ along blood vessels or lymphatic vessels.

Since metastasis cancer shares biochemical characteristics with primary cancer, treatment methods that are similar to those applied to primary cancer are applied to metastatic cancer regardless of the location where the metastatic cancer is generated. Accordingly, in selecting the optimal therapeutic agent or treatment method, the stage of specifying the primary site of cancer needs to be preceded.

For most metastatic cancers, the primary site may be specified through pathological examination of a sample, but in some cases, the primary site may not be specified even after immunohistochemical staining, molecular genetic testing, and tumor marker testing are performed. This is called Carcinoma of Unknown Primary (CUP).

It is estimated that about 3-5% of all cancer patients have cancer of unknown primary site. As described above, it is reported that the prognosis is very poor compared to general cancer patients because an appropriate therapeutic agent or treatment method may not be specified.

On the other hand, recently, attempts have been made to specify the primary site of cancer of unknown primary site by using the gene expression pattern of a sample collected from the lesion site. According to the method described above, in the state in which specific gene expression patterns appearing for each primary cancer are learned, the primary site may be specified by comparing the learned specific gene expression pattern for each primary cancer with the gene expression information of the sample acquired from the lesion site.

The diagnosis method using gene expression pattern information has the advantage of relatively accurately diagnosing the primary site when accurate gene expression patterns are acquired from metastatic cancer tissue, but gene expression information is mixed with gene expression patterns derived from the primary cancer and gene expression patterns derived from the metastasized tissue itself. Accordingly, it is difficult to acquire an accurate gene expression pattern in the sample.

Accordingly, the need for a method for diagnosing cancer of unknown primary site using a new form of artificial intelligence that may increase the accuracy of diagnosis has emerged.

### SUMMARY

The present invention has been devised to obviate the above limitation, and to provide a method for diagnosing cancer of unknown primary site using artificial intelligence capable of specifying a primary site using gene expression pattern information of a sample acquired from metastatic cancer tissue.

An aspect of the present invention is directed to providing a method for diagnosing cancer of unknown primary site using artificial intelligence capable of isolating only gene expression patterns derived from primary cancer from samples acquired from metastatic cancer tissue.

The aspect of the present invention is not limited to those mentioned above, and other aspects not mentioned herein will be clearly understood by those skilled in the art from the following description.

The method for diagnosing cancer of unknown primary site using artificial intelligence according to an embodiment of the present invention includes: generating gene expression pattern information of a sample collected from tissue in which metastatic cancer has occurred; removing gene expression pattern information derived from pre-learned tissue from the gene expression pattern information of the sample collected from the tissue in which the metastatic cancer has occurred; comparing the gene expression pattern information from which the gene expression pattern information derived from the tissue has been removed with pre-learned gene expression pattern information for each cancer type; and specifying a primary site of the sample collected from the tissue in which the metastatic cancer has occurred.

According to an embodiment of the present invention, the sample collected from the tissue in which the metastatic cancer has occurred may include normal tissue and cancer tissue of an organ in which the metastatic cancer has occurred.

According to an embodiment of the present invention, the gene expression pattern information derived from the tissue may be specific gene expression pattern information expressed in normal tissue of an organ.

According to an embodiment of the present invention, the gene expression pattern information for each cancer type may be specific gene expression pattern information expressed in cancer tissue of which the primary site is specified.

According to an embodiment of the present invention, the removing of the gene expression pattern information derived from the pre-learned tissue from the gene expression pattern information of the sample collected from the tissue in which the metastatic cancer has occurred may include: converting the gene expression pattern information of the sample collected from the tissue in which the metastatic cancer has occurred into a first vector; converting the gene expression pattern information derived from the tissue into a second vector; and performing a difference calculation of the second vector with respect to the first vector.

According to an embodiment of the present invention, the specifying of the primary site of the sample collected from the tissue in which the metastatic cancer has occurred may include specifying at least one of a plurality of pre-learned primary sites.

According to an embodiment of the present invention, the specifying of the primary site of the sample collected from the tissue in which the metastatic cancer has occurred may include outputting a probability value for each primary site.

According to an embodiment of the present invention, the gene expression pattern information of the sample collected from the tissue in which the metastatic cancer has occurred, the gene expression pattern information derived from the tissue, and the gene expression pattern information from which the gene expression pattern information derived from the tissue has been removed may be RNA sequence information.

According to an embodiment of the present invention, the RNA sequence information may be mRNA sequence information.

According to another embodiment of the present invention, an apparatus for diagnosing cancer of unknown primary site using artificial intelligence includes: a memory storing one or more instructions; and a processor, by executing one or more of the stored instructions, performing an operation of generating gene expression pattern information of a sample collected from tissue in which metastatic cancer has occurred, an operation of removing gene expression pattern information derived from pre-learned tissue from the gene expression pattern information of the sample collected from the tissue in which the metastatic cancer has occurred, an operation of comparing the gene expression pattern information from which the gene expression pattern information derived from the tissue has been removed with pre-learned gene expression pattern information for each cancer type, and an operation of specifying a primary site of the sample collected from the tissue in which the metastatic cancer has occurred.

According to the aforementioned method for diagnosing cancer of unknown primary site, in specifying the primary site of cancer of unknown primary site using a gene expression pattern, it is possible to exclude gene expression patterns attributed to the tissue where metastatic cancer is generated, thus further improving the accuracy of diagnosis.

### BRIEF DESCRIPTION OF THE DRAWINGS

FIG. 1 is an exemplary diagram illustrating an apparatus 100 for diagnosing cancer of unknown primary site using artificial intelligence according to an embodiment of the present invention.
FIG. 2 is a flowchart illustrating a method for diagnosing cancer of unknown primary site using artificial intelligence according to an embodiment of the present invention.
FIG. 3 is a diagram illustrating a gene expression pattern of a sample acquired from metastatic cancer tissue according to an embodiment of the present invention.
FIG. 4 is a diagram illustrating gene expression pattern information learned by an apparatus for diagnosing cancer of unknown primary site using artificial intelligence according to an embodiment of the present invention.
FIG. 5 is a conceptual diagram illustrating a method for specifying a primary site of cancer of unknown primary site by acquiring gene expression pattern information derived from cancer occurring at the primary site according to an embodiment of the present invention.
FIG. 6 is a diagram illustrating a method of performing a difference calculation between gene expression patterns represented by feature vectors according to an embodiment of the present invention.
FIG. 7 is a diagram visualizing pre-learned gene expression pattern information for each tissue according to an embodiment of the present invention.
FIGS. 8 and 9 are diagrams for comparing gene expression patterns before and after excluding gene expression patterns derived from the tissue in which the metastatic cancer has occurred.

### DESCRIPTION OF EXEMPLARY EMBODIMENTS

Hereinafter, preferred embodiments of the present disclosure will be described in detail with reference to the accompanying drawings. The advantages and features of the present disclosure and methods of achieving the same will be apparent from the embodiments that will be described in detail with reference to the accompanying drawings. It should be noted, however, that the technical ideas of the present disclosure are not limited to the following embodiments, and may be implemented in various different forms. Rather the embodiments are provided so that the technical ideas of the present disclosure will be thorough and complete and will fully convey the scope of the present disclosure to those skilled in the technical field to which the present disclosure pertains. It is to be noted that the technical ideas of the present disclosure are defined only by the claims.

In adding reference numerals for elements in each drawing, it should be noted that like reference numerals designate like elements wherever possible even though elements are shown in other drawings. Furthermore, in describing the present disclosure, a detailed description of the related known functions and constructions will be omitted if it is deemed to make the gist of the present disclosure vague.

Unless otherwise defined, all terms including technical and scientific terms used herein have the same meaning as commonly understood by those skilled in the technical field to which the present disclosure pertains. It will be understood that terms, such as those defined in commonly used dictionaries, should be interpreted as having a meaning that is consistent with their meaning in the context of the relevant art and will not be interpreted in an idealized or overly formal sense unless expressly so defined herein. Terms used in the specification are used to describe embodiments of the present disclosure and are not intended to limit the scope of the present disclosure. In the specification, the terms in singular form may include plural forms unless otherwise specified.

In addition, in the description of the components of the embodiment of the present disclosure, the terms such as first, second, A, B, (a), and (b) may be used. These terms are merely used to distinguish the components from other components, and do not delimit an essence, an order or a sequence of the corresponding components. When it is described that a component is "connected", "coupled", or "jointed" to another component, the description may include not only being directly connected, coupled or joined to the other component but also being "connected" "coupled" or "joined" by another component between the component and the other component.

The terms "comprises" and/or "comprising" used herein do not preclude the presence or addition of one or more other components, steps, operations, and/or elements, in addition to the mentioned components, steps, operations, and/or elements.

Prior to the description of the present disclosure, some terms used in the following embodiments will be clarified.

In the following examples, gene expression pattern information means various types of data related to gene expression. For example, it may mean data on transcripts, proteomes, and the like. In addition, it may include data on DNA sequence information, RNA sequence information, RNA or DNA expression level, expression ratio, expression position, expression distribution, etc.

Hereinafter, various embodiments of the present disclosure will be described in detail with reference to the accompanying drawings.

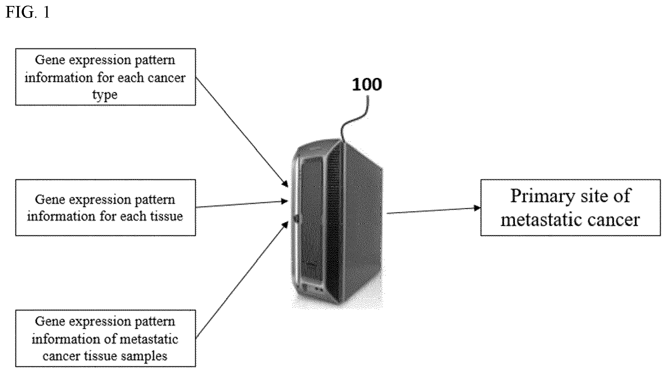

FIG. 1 is an exemplary diagram illustrating an apparatus 100 for diagnosing cancer of unknown primary site using artificial intelligence according to an embodiment of the present invention.

As illustrated in FIG. 1, the apparatus 100 for diagnosing cancer of unknown primary site using artificial intelligence according to an embodiment of the present invention receives gene expression pattern information for each cancer type and gene expression pattern information for each tissue as learning data, and receives gene expression pattern information of samples acquired from metastatic cancer tissue as query data.

The gene expression pattern information for each cancer type refers to gene expression pattern information of a cancer tissue of which a primary site is specified. For example, the term refers to a gene expression pattern of a sample acquired from a cancer tissue whose primary site is the liver, a gene expression pattern of a sample acquired from a cancer tissue whose primary site is the lung, and the like. According to an embodiment of the present invention, another gene expression pattern may be RNA sequence information, more specifically, mRNA sequence information.

The gene expression pattern information for each tissue refers to gene expression pattern information derived from the tissue of an organ. Different combinations of genes are expressed in various organs configuring the body, so gene expression pattern information is different for each tissue. The gene expression pattern for each tissue may also be RNA sequence information, more specifically, mRNA sequence information.

On the other hand, receiving gene expression pattern information for each cancer type and gene expression pattern information for each tissue as learning data means receiving a plurality of pieces of gene expression pattern information in which the primary site is labeled.

The gene expression pattern information labeled with the primary site according to an embodiment of the present invention may be data crawled from databases such as GEO (Gene Expression Omnibus), Array Express, TCGA (The Cancer Genome Atlas Program), ICGs (Iterative Clustering and Guide-Gene selection), and GTEx (Genotype Tissue Expression).

The apparatus 100 for diagnosing cancer of unknown primary site using artificial intelligence according to an embodiment of the present invention uses labeled learning data to derive the characteristics of the gene expression pattern of the cancer of which primary site is specified and the gene expression pattern derived from the tissue in which metastatic cancer has occurred.

In a state in which learning is completed, when gene expression pattern information of a sample acquired from metastatic cancer tissue is input as query data, the primary site of metastatic cancer is output as a result value.

The result value according to an embodiment of the present invention may be output in the form of specifying at least one of a plurality of learned primary sites or outputting a probability value for each primary site.

For example, when the gene expression pattern information of a sample acquired from metastatic cancer tissue is input as query data, the primary site of the metastatic cancer is output in the form of "liver", or the probability that the primary site is "liver" may be output as X% and the probability that the primary site is "lung" may be output as Y%, and the like.

Although FIG. 1 illustrates an example in which the apparatus 100 for diagnosing cancer of unknown primary site using artificial intelligence is implemented as a single computing device, the apparatus 100 for diagnosing cancer of unknown primary site using artificial intelligence may be implemented as a plurality of physically or logically divided computing devices. In this connection, a first function of the apparatus 100 for diagnosing cancer of unknown primary site using artificial intelligence may be implemented in a first computing device, and a second function may be implemented in a second computing device. Alternatively, a specific function of the apparatus 100 for diagnosing cancer of unknown primary site using artificial intelligence may also be implemented in a plurality of computing devices.

Hereinafter, a method of outputting the primary site of cancer of unknown primary site, which is output data, using the input data will be described in detail.

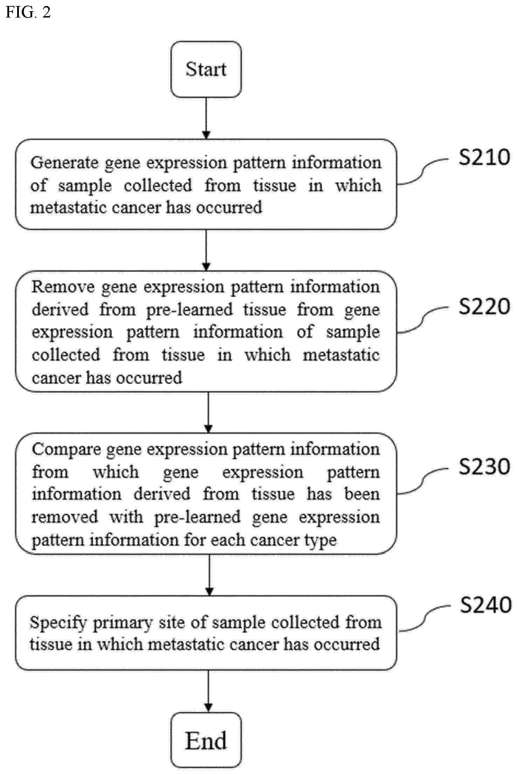

FIG. 2 is a flowchart illustrating a method for diagnosing cancer of unknown primary site using artificial intelligence according to an embodiment of the present invention.

In the following, for convenience of description, the subject performing each stage is omitted. However, those skilled in the art to which the present invention pertains may understand that each stage is performed in the apparatus 100 for diagnosing cancer of unknown primary site using artificial intelligence.

First, gene expression pattern information of a sample collected from a tissue in which metastatic cancer has occurred is generated (S210). According to an embodiment of the present invention, a sample collected from a tissue in which the metastatic cancer has occurred may include normal tissue and cancer tissue of an organ in which the metastatic cancer has occurred.

In addition, the gene expression pattern information may be acquired by performing transcriptional genome sequencing on the sample. Specifically, the gene expression pattern information according to an embodiment of the present invention may be RNA sequence information, more specifically, mRNA sequence information.

Thereafter, the gene expression pattern information derived from the pre-learned tissue is removed from the gene expression pattern information of the sample collected from the tissue in which metastatic cancer has occurred (S220).

Here, the gene expression pattern information derived from the tissue refers to gene expression pattern information specifically expressed in a tissue in which metastatic cancer has occurred. For example, when the organ in which metastatic cancer has occurred is "lung," the term refers to specific gene expression pattern information expressed in "lung."

When the gene expression pattern information derived from the tissue is removed from gene expression pattern information of the sample collected from the tissue in which the metastatic cancer has occurred, only gene expression pattern information derived from a primary cancer remains.

Accordingly, when the gene expression pattern information from which the gene expression pattern information derived from an organ tissue in which metastatic cancer has occurred is removed and the pre-learned gene expression pattern information for each cancer type are compared (S530), the primary site of metastatic cancer may be specified (S540).

Hereinafter, a method for specifying a primary site using gene expression pattern information of a sample will be described in detail.

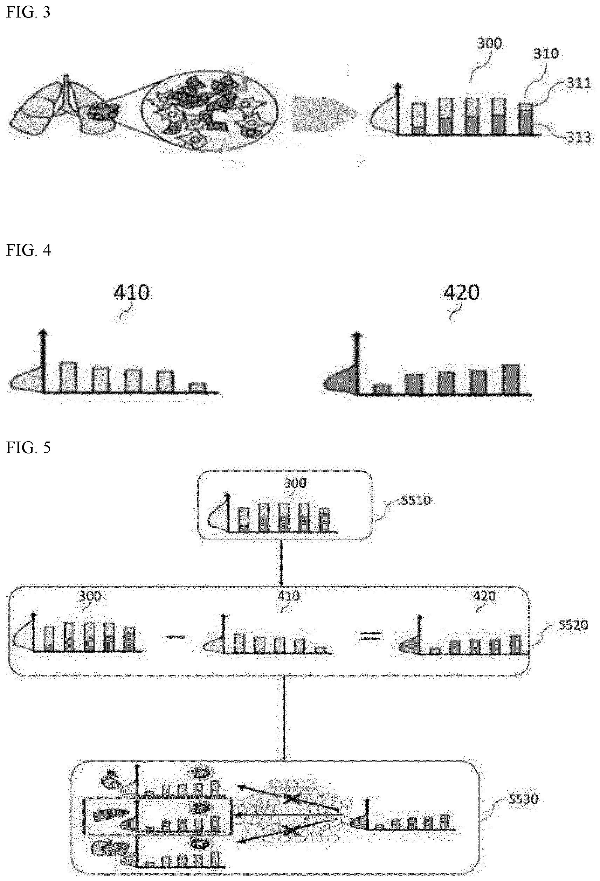

FIG. 3 is a diagram illustrating a gene expression pattern of a sample acquired from metastatic cancer tissue according to an embodiment of the present invention.

In order to acquire gene expression patterns in metastatic cancer tissue, tissue from an organ in which metastatic cancer has occurred are acquired.

Fragments of tissues according to an embodiment of the present invention may be collected in a form of cutting into a predetermined size to include cancer tissue of unknown primary site and normal tissue of an organ.

When transcriptional genome sequencing is performed on the sample collected by the aforementioned method, gene expression pattern information 300 of the sample acquired from the metastatic cancer tissue may be acquired.

According to an embodiment of the present invention, the gene expression pattern information 300 of a sample acquired from metastatic cancer tissue may be expressed as an expression level for each gene. Specifically, the x-axis of the gene expression pattern information 300 of a sample acquired from metastatic cancer tissue illustrated in FIG. 3 means a plurality of discretely arranged genes, and the y-axis means the relative or absolute expression level of the gene.

As illustrated in FIG. 3, in gene expression pattern information 310 acquired from metastatic cancer tissue, gene expression patterns derived from cancers generated in the primary site and gene expression patterns derived from organ tissue in which metastatic cancer has occurred are mixed.

For example, when the organ in which metastatic cancer has occurred illustrated in FIG. 3 is "lung," among the gene expression pattern information collected from the lesion site, the first gene expression information 310 is mixed with a gene expression pattern 311 derived from the lung, which is an organ tissue in which metastatic cancer has occurred and a gene expression pattern 313 derived from a cancer occurring in a primary site.

When the gene expression pattern information 310 acquired from metastatic cancer tissue is directly compared with the gene expression pattern information for each cancer type with a specific primary site, the gene expression pattern derived from the organ tissue in which metastatic cancer has occurred acts as noise, thus making only the gene expression pattern information impossible to accurately specify the primary site.

Accordingly, from the gene expression pattern information 310 acquired from metastatic cancer tissue, the gene expression pattern information 313 derived from organ tissue in which metastatic cancer has occurred, acting as noise, needs to be excluded.

Hereinafter, a method of extracting a gene expression pattern derived from a cancer generated in a primary site from gene expression pattern information of a sample acquired from metastatic cancer tissue will be described.

FIG. 4 is a diagram illustrating gene expression pattern information learned by an apparatus for diagnosing cancer of unknown primary site using artificial intelligence according to an embodiment of the present invention.

The apparatus 100 for diagnosing cancer of unknown primary site according to an embodiment of the present invention may store gene expression pattern information 410 of tissue learned through a plurality of pieces of learning data and gene expression pattern information 420 for each cancer type.

The gene expression pattern information 410 of a tissue refers to a specific gene expression pattern appearing in a normal tissue of an organ. For example, the term refers to a gene expression pattern specifically appearing in a normal tissue of the lung, a gene expression pattern specifically appearing in a normal tissue of the liver, and the like.

The gene expression pattern information 420 for each caner type refers to a gene expression pattern specifically appearing in a cancer tissue in which a primary site is specified. For example, the term refers to a gene expression pattern specifically appearing in a cancer tissue whose primary site is the lung, and a gene expression pattern specifically appearing in a cancer tissue whose primary site is the liver.

Using the learned data, it is possible to acquire gene expression pattern information derived from cancer occurring in the primary site from gene expression pattern information of a sample acquired from metastatic cancer tissue.

FIG. 5 is a conceptual diagram illustrating a method for specifying a primary site of cancer of unknown primary site by acquiring gene expression pattern information derived from cancer occurring at the primary site according to an embodiment of the present invention.

The apparatus 100 for diagnosing cancer of unknown primary site using artificial intelligence according to an embodiment of the present invention generates the gene expression pattern information 300 of metastatic cancer tissue through sequencing of samples collected from metastatic cancer tissue (S510).

The gene expression pattern information 300 of metastatic cancer tissue according to an embodiment of the present invention may be expression levels of genes specified by mRNA sequence information.

Thereafter, a difference calculation is performed between the gene expression pattern information 300 of the metastatic cancer tissue and the gene expression pattern information 410 of the pre-learned tissue to calculate the gene expression pattern information 420 for each cancer type (S520). Herein, the gene expression pattern information 410 of a pre-learned tissue refers to a normal tissue of an organ in which a pre-learned metastatic cancer has occurred, as described above.

The gene expression pattern information 300 of metastatic cancer tissue and the gene expression pattern information 410 of tissue according to an embodiment of the present invention may be displayed as a feature vector on a multi-dimensional space.

Accordingly, the difference calculation between the gene expression pattern information 300 of metastatic cancer tissue and the gene expression pattern information 410 of pre-learned tissue is made based on the difference calculation between the gene expression feature vector of metastatic cancer tissue and the gene expression feature vector of the tissue.

The pure gene expression pattern information 420 for each cancer type excluding the gene expression pattern information 410 derived from the tissue in which metastatic tissue has occurred may be acquired through the aforementioned difference calculation.

When the gene expression pattern information 420 for each cancer type is acquired through the difference calculation, the similarity with the pre-learned gene expression pattern information for each cancer type is calculated to specify the primary site of the gene expression pattern with the highest similarity to the primary site of the metastatic cancer (S530).

For example, when the gene expression pattern information 420 for each cancer type acquired through the difference calculation is most similar to the gene expression pattern of cancer tissue whose primary site is the liver, the primary site of metastatic cancer is specified as the liver.

Similarly, when the gene expression pattern information 420 for each cancer type acquired through the difference calculation is most similar to the gene expression pattern of cancer tissue whose primary site is the lung, the primary site of metastatic cancer is specified as the lung.

According to the aforementioned method for diagnosing cancer of unknown primary site, in specifying the primary site of cancer of unknown primary site using a gene expression pattern, it is possible to exclude gene expression patterns derived from tissues in which metastatic cancer has occurred, thus further increasing the accuracy of diagnosis.

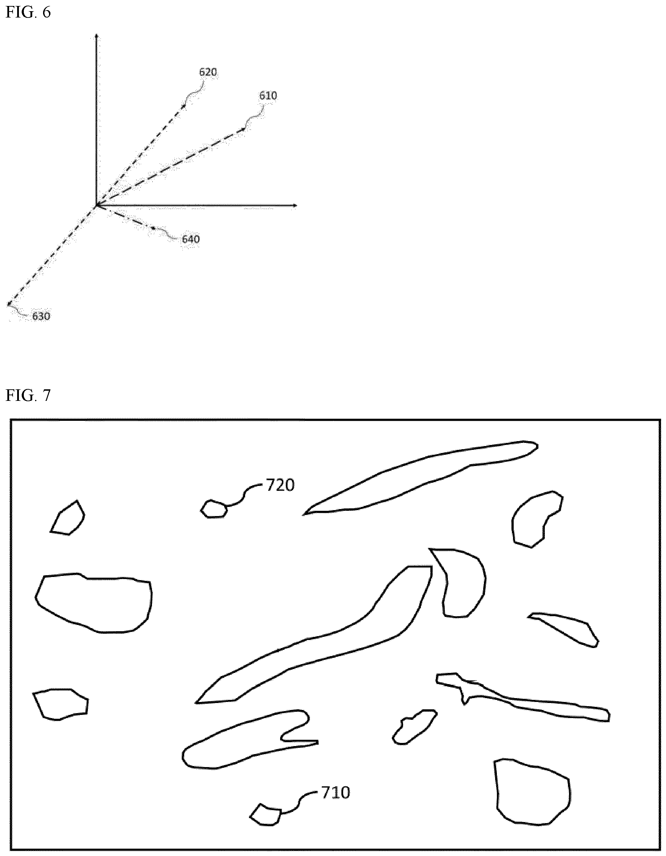

FIG. 6 is a diagram illustrating a method of performing a difference calculation between gene expression patterns represented by feature vectors according to an embodiment of the present invention.

FIG. 6 illustrates gene expression pattern information of metastatic cancer tissue expressed as a vector, gene expression pattern information of tissue, and gene expression pattern information for each cancer type.

Specifically, a first vector 610 is the gene expression pattern information 300 of metastatic cancer tissue described in FIG. 5, and a second vector 620 is the gene expression pattern information of tissue.

In FIG. 6, the first vector 610 and the second vector 620 have been described as vectors located on a two-dimensional space as an example, but may actually be vectors displayed on a multi-dimensional space.

Alternatively, the vectors displayed on a multi-dimensional space may be converted into vectors displayed on a 2-dimensional space by applying a dimensionality reduction technique. Examples of dimensionality reduction techniques include Uniform Manifold Approximation and Projection (UMAP), Locally Linear Embedding (LLE), Multi-Dimensional Scaling (MDS), Principal Component Analysis (PCA), Singular Value Decomposition (SVD), and Non-negative Matrix Factorization (NMF), but is not limited thereto, and dimensionality reduction techniques widely known in the art may be applied without limitation.

In order to calculate a third vector 640 corresponding to the gene expression pattern information for each cancer type from the first vector 610 corresponding to the gene expression pattern information of metastatic cancer tissue and the second vector 620 corresponding to the gene expression pattern information of tissue, a difference calculation is performed on the first vector 610 and the second vector 620.

The difference calculation according to an embodiment of the present invention decomposes the first vector 610 and the second vector 620 for each component (for example, an x-axis component and a y-axis component), and then performs a difference calculation between the same components.

Alternatively, after calculating an inverse vector 630 of the second vector 620 as an intermediate vector, a third vector 640 may be calculated by adding the first vector 610 and the inverse vector 630.

However, a specific method of performing a difference calculation between the first vector 610 and the second vector 620 is not limited thereto, and other general-purpose algorithms may be applied.

FIG. 7 is a diagram visualizing pre-learned gene expression pattern information for each tissue according to an embodiment of the present invention.

Different combinations of genes are expressed in various organs constituting the body, so gene expression pattern information is different for each tissue. In other words, the normal tissue of an organ that does not contain cancer cells has a gene pattern that is specifically expressed for each organ type.

Accordingly, data as illustrated in FIG. 7 may be obtained by learning and visualizing gene expression patterns of samples collected from normal tissues. In FIG. 7, for convenience of understanding, gene expression patterns are visualized on a two-dimensional space as an example, but gene expression patterns may be arranged on a multi-dimensional space.

A cluster illustrated in FIG. 7 refers to a pattern of genes expressed in the same tissue.

For example, a first cluster 710 refers to gene expression patterns in normal tissues of the liver, and a second cluster 720 refers to gene expression patterns in normal tissues of salivary glands.

As described above, the learned gene expression pattern for each tissue illustrated in FIG. 7 may be used to remove gene expression patterns derived from the tissue itself, which acts as noise in the sample collected from the tissue in which metastatic cancer has occurred.

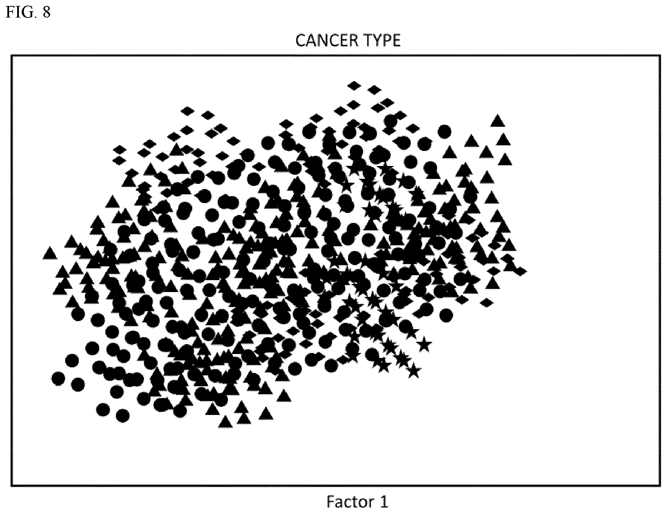

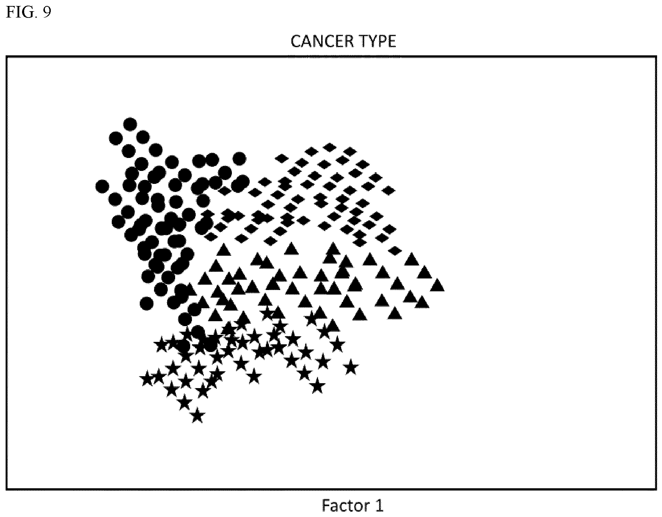

FIGS. 8 and 9 are diagrams for comparing gene expression patterns before and after excluding gene expression patterns derived from the tissue in which the metastatic cancer has occurred.

FIG. 8 illustrates the gene expression pattern of the sample collected from metastatic cancer tissue with a specified primary site. In other words, in the gene expression pattern illustrated in FIG. 8, a gene expression pattern derived from the tissue in which metastatic cancer has occurred and a gene expression pattern derived from a primary cancer are mixed.

Cancers with the same primary site are displayed in the same color. As shown in FIG. 8, markers of various colors are mixed, making it difficult to specify the primary site using only gene expression patterns.

For example, since a plurality of markers having different colors are located at specific positions in a two-dimensional space, it is not possible to clearly specify the primary site of cancer occurred in a corresponding sample.

FIG. 9 illustrates a gene expression pattern in which the gene expression pattern derived from the tissue itself in which metastatic cancer has occurred is removed. In other words, the markers illustrated in FIG. 9 refer to gene expression patterns derived from the primary cancer itself. In FIG. 9, since markers having the same color are clustered, the location of the primary cancer may be specified only by the gene expression pattern.

Accordingly, a primary site can be clearly specified from the gene expression pattern of a sample collected from metastatic cancer tissue.

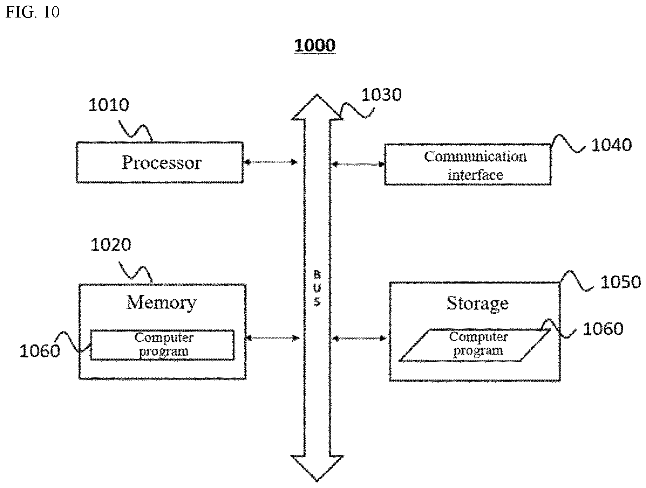

FIG. 10 is a functional block diagram illustrating an apparatus for diagnosing cancer of unknown primary site using artificial intelligence according to another embodiment of the present invention.

As illustrated in FIG. 10, an apparatus 1000 for diagnosing cancer of unknown primary site using artificial intelligence includes one or more processors 1010, a memory 1020 that loads a computer program performed by the processor 1010, a bus 1030, a communication interface 1040, and a storage 1050 that stores a computer program 1060.

However, FIG. 10 illustrates only the constituents related to the embodiment of the present disclosure. Accordingly, it should be understood by those skilled in the art to which the present disclosure pertains that other general-purpose constituents may be further included in addition to the constituents illustrated in FIG. 10. In other words, the apparatus 1000 for diagnosing cancer of unknown primary site using artificial intelligence may further include various constituents in addition to the constituents illustrated in FIG. 10. Alternatively, the apparatus 1000 for diagnosing cancer of unknown primary site using artificial intelligence may be configured by excluding some of the constituents illustrated in FIG. 10.

The processor 1010 may control the overall operation of each configuration of the apparatus 1000 for diagnosing cancer of unknown primary site using artificial intelligence. The processor 1010 may be configured by including at least one of a Central Processing Unit (CPU), a Micro-Processor Unit (MPU), a Micro-Controller Unit (MCU), a Graphics Processing Unit (GPU), or any arbitrary type of processor well known to the technical field of the present disclosure. In addition, the processor 1010 may perform calculations on at least one application or program for executing the methods/operations according to the embodiments of the present disclosure. The apparatus 1000 for diagnosing cancer of unknown primary site using artificial intelligence may be provided with one or more processors.

The memory 1020 may store various pieces of data, instructions, and/or information. The memory 1020 may load one or more computer programs 1060 from the storage 1050 to execute the methods according to the embodiments of the present disclosure. The memory 1020 may be implemented using a volatile memory such as RAM, but is not limited thereto.

The bus 1030 may provide a communication function between the constituents of the apparatus 1000 for diagnosing cancer of unknown primary site using artificial intelligence. The bus 1030 may be implemented using various types of buses such as address bus, data bus, and control bus.

The communication interface 1040 may support wired and wireless Internet communication of the apparatus 1000 for diagnosing cancer of unknown primary site using artificial intelligence. In addition, the communication interface 1040 may support various communication schemes in addition to Internet communication. To this end, the communication interface 1040 may be configured to include a communication module well known in the technical field of the present disclosure. In some embodiments, the communication interface 1040 may be omitted.

The storage 125 may store the one or more programs 1060 non-temporarily. The storage 125 may be configured to include non-volatile memory such as a Read-Only Memory (ROM), an Erasable Programmable ROM (EPROM), an Electrically Erasable Programmable ROM (EEPROM), and a flash memory; a hard disk; a removable disk; or any type of computer-readable recording medium well known in the technical field to which the present disclosure pertains.

The computer program 1060, when loaded into the memory 1020, may include one or more instructions that instruct the processor 1010 to perform the methods/operations according to various embodiments of the present disclosure. In other words, by executing the one or more instructions, the processor 1010 may perform the methods/operations according to various embodiments of the present disclosure.

For example, the computer program 1060 may include instructions that instruct the processor to perform: an operation of generating gene expression pattern information of a sample collected from tissue in which metastatic cancer has occurred; an operation of removing gene expression pattern information derived from pre-learned tissue from the gene expression pattern information of the sample collected from the tissue in which the metastatic cancer has occurred; an operation of comparing the gene expression pattern information from which the gene expression pattern information derived from the tissue has been removed with pre-learned gene expression pattern information for each cancer type; and an operation of specifying a primary site of the sample collected from the tissue in which the metastatic cancer has occurred.

The technical spirit of the present disclosure, described so far with reference to FIGS. 1 to 10 , may be implemented in computer-readable code on a computer-readable medium. The computer-readable recording medium may include, for example, a removable recording medium (CD, DVD, Blu-ray Disc, USB storage device, removable hard disk), or a stationary recording medium (ROM, RAM, or a built-in computer hard disk). The computer program recorded in a computer-readable recording medium may be transmitted to a different computing device through a network such as the Internet and installed in the different computing device, thereby being used in the different computing device.

In the above, just because all the constituents configuring an embodiment of the present disclosure are combined into one or operate in combination with each other does not mean that the technical spirit of the present disclosure are necessarily limited to the embodiment. In other words, as long as being within the target scope of the present disclosure, all the constituents may operate by being selectively integrated into one or more combinations.

Although the operations are illustrated in a particular order in the figure, it should not be understood that the operations have to be performed in the specific order or in the sequential order according to which the operations are illustrated or that a desired result may be achieved only when all the illustrated operations are executed. In certain situations, multitasking and parallel processing may be advantageous. Moreover, separation into various configurations in the embodiments described above should not be understood as being required necessarily, and the program components and systems described above may generally be integrated into a single software product or packaged into multiple software products.

So far, although the embodiments of the present disclosure have been described with reference to appended drawings, it should be understood by those skilled in the art to which the present disclosure pertains that the present disclosure may be embodied in other specific forms without changing the technical principles or essential characteristics of the present disclosure. Therefore, the embodiments described above should be regarded as being illustrative rather than restrictive in every aspect. The scope of protection of the present disclosure should be determined on the basis of the descriptions in the appended claims, and all technical ideas within the scope of equivalents thereof should be construed as being included in the scope of right of the technical ideas defined in the present disclosure.

## Claims

1. A method for diagnosing cancer of unknown primary site using artificial intelligence, wherein the method comprises:
generating gene expression pattern information of a sample collected from tissue in which metastatic cancer has occurred;
removing gene expression pattern information derived from pre-learned tissue from the gene expression pattern information of the sample collected from the tissue in which the metastatic cancer has occurred;
comparing the gene expression pattern information from which the gene expression pattern information derived from the tissue has been removed with pre-learned gene expression pattern information for each cancer type; and
specifying a primary site of the sample collected from the tissue in which the metastatic cancer has occurred.

2. The method of claim 1, wherein the sample collected from the tissue in which the metastatic cancer has occurred comprises normal tissue and cancer tissue of an organ in which the metastatic cancer has occurred.

3. The method of claim 1, wherein the gene expression pattern information derived from the tissue is specific gene expression pattern information expressed in normal tissue of an organ.

4. The method of claim 1, wherein the gene expression pattern information for each cancer type is specific gene expression pattern information expressed in cancer tissue of which the primary site is specified.

5. The method of claim 1, wherein the removing of the gene expression pattern information derived from the pre-learned tissue from the gene expression pattern information of the sample collected from the tissue in which the metastatic cancer has occurred comprises:
converting the gene expression pattern information of the sample collected from the tissue in which the metastatic cancer has occurred into a first vector;
converting the gene expression pattern information derived from the tissue into a second vector; and
performing a difference calculation of the second vector with respect to the first vector.

6. The method of claim 1, wherein the specifying of the primary site of the sample collected from the tissue in which the metastatic cancer has occurred comprises specifying at least one of a plurality of pre-learned primary sites.

7. The method of claim 1, wherein the specifying of the primary site of the sample collected from the tissue in which the metastatic cancer has occurred comprises outputting a probability value for each primary site.

8. The method of claim 1, wherein the gene expression pattern information of the sample collected from the tissue in which the metastatic cancer has occurred, the gene expression pattern information derived from the tissue, and the gene expression pattern information from which the gene expression pattern information derived from the tissue has been removed are RNA sequence information.

9. The method of claim 8, wherein the RNA sequence information is mRNA sequence information.

10. An apparatus for diagnosing cancer of unknown primary site using artificial intelligence, the apparatus comprising:
a memory storing one or more instructions; and
a processor, by executing one or more of the stored instructions, performing:
an operation of generating gene expression pattern information of a sample collected from tissue in which metastatic cancer has occurred;
an operation of removing gene expression pattern information derived from pre-learned tissue from the gene expression pattern information of the sample collected from the tissue in which the metastatic cancer has occurred;
an operation of comparing the gene expression pattern information from which the gene expression pattern information derived from the tissue has been removed with pre-learned gene expression pattern information for each cancer type; and
an operation of specifying a primary site of the sample collected from the tissue in which the metastatic cancer has occurred.
